# FASCICULE DE BREVET EUROPEEN

(11) **EP 1 673 455 B1**
(45) Date de publication et mention de la délivrance du brevet: **05.03.2008**
(21) Numéro de dépôt: 04791526.9
(22) Date de dépôt: 13.10.2004
(51) Int. Cl.: C12N 15/10

(54) **METHODE D'EXTRACTION D'ACIDES NUCLEIQUES**
NUKLEINSÄUREEXTRAKTIONSVERFAHREN
NUCLEIC ACID EXTRACTION METHOD

(30) Priorité: 15.10.2003 FR 0312049
(43) Date de publication de la demande: 28.06.2006
(73) Titulaire: Bertin Technologies S.A., 78180 Montigny-le-Bretonneux (FR)
(72) Inventeur: MARON, Pierre-Alain, F-21220 FIXIN (FR); LEJON, David, F-21000 Dijon (FR); BIZET, Karine, F-92100 BOULOGNE (FR); CARVALHO, Esmeralda, F-78790 Arnouville (FR)
(74) Mandataire: Ramey, Daniel
(86) Numéro de dépôt international: PCT/FR2004/002613
(87) Numéro de publication internationale: WO 2005/038025

(56) Documents cités:
- EP-A- 0 795 602
- KUSKE C R ET AL: "Small-scale DNA sample preparation method for field PCR detection of microbial cells and spores in soil" APPLIED AND ENVIRONMENTAL MICROBIOLOGY, WASHINGTON,DC, US, vol. 64, no. 7, juillet 1998 (1998-07), pages 2463-2472, XP002226433 ISSN: 0099-2240
- GRIFFIN D W ET AL: "A rapid and efficient assay for extracting DNA from fungi" LETTERS IN APPLIED MICROBIOLOGY, OXFORD, GB, vol. 34, no. 3, 2002, pages 210-214, XP002290777
- MILLER DN ET AL: "Evaluation and optimization of DNA extraction and purification procedures for soil and sediment samples" APPLIED AND ENVIRONMENTAL MICROBIOLOGY, WASHINGTON,DC, US, vol. 65, no. 11, 1999, pages 4715-4724, XP002148457 ISSN: 0099-2240
- MORE M I ET AL: "QUANTITATIVE CELL LYSIS OF INDIGENOUS MICROORGANISMS AND RAPID EXTRACTION OF MICROBIAL DNA FROM SEDIMENT" APPLIED AND ENVIRONMENTAL MICROBIOLOGY, WASHINGTON,DC, US, vol. 60, no. 5, 1 mai 1994 (1994-05-01), pages 1572-1580, XP000574303 ISSN: 0099-2240
- ODUMERU J ET AL: "Use of the bead beater for preparation of Mycobacterium paratuberculosis template DNA in milk." CANADIAN JOURNAL OF VETERINARY RESEARCH = REVUE CANADIENNE DE RECHERCHE VETERINAIRE. OCT 2001, vol. 65, no. 4, octobre 2001 (2001-10), pages 201-205, XP008044069 ISSN: 0830-9000

## Description

La présente invention porte sur une méthode d'extraction d'acides nucléiques de micro-organismes, et son utilisation dans un procédé d'analyse d'une population microbienne d'un environnement donné, notamment d'une population de micro-organismes prélevés dans l'air. En particulier, l'invention porte sur une méthode d'extraction comprenant la mise en oeuvre successive des trois étapes de lyse suivantes dans un ordre quelconque :
i. une lyse chimique des micro-organismes comprenant la mise au contact des micro-organismes avec une solution de lyse contenant un détergent ;
ii. une lyse par choc thermique comprenant l'incubation de l'extrait à une température inférieure à 0°C, suivie immédiatement de l'incubation de l'extrait à une température d'au moins 95°C, de préférence environ 100°C ; et,
iii. une lyse mécanique comprenant l'agitation de l'extrait en présence de billes.

Les changements climatiques ou les changements de l'écosystème associés à l'urbanisme, à l'agriculture ou à l'industrie sont généralement corrélés à des modifications de la composition de la flore microbienne dans l'environnement. Il est donc possible, en théorie, de détecter des modifications anormales de l'écosystème par l'analyse de l'évolution de la composition d'une population microbienne d'un environnement donné au cours du temps.

Un tel suivi serait également utile pour détecter la présence anormale d'agents pathogènes dans l'environnement, par exemple liés à leur dissémination dans l'air par des armes biologiques.

Cependant, l'impossibilité de cultiver la plupart des micro-organismes prélevées dans l'environnement tels que le sol ou l'air constitue un obstacle majeur pour l'analyse de l'écologie microbienne et de sa diversité.

L'utilisation de techniques de biologie moléculaire permet de surmonter cet obstacle, par l'analyse directe de séquences d'acides nucléiques spécifiques d'espèces de micro-organismes contenus dans l'échantillon analysé, en s'affranchissant des étapes de culture.

En particulier, la technique RISA (Ribosomal Intergenic Spacer Amplification), fondée sur l'analyse du polymorphisme de longueur de la région intergénique 16S/23S de l'ARN ribosomique, permet de caractériser des populations de micro-organismes et de les comparer entre elles (Ranjard et al., oct 2001, Applied and Environmental Microbiology, pp 4479-4487, Ranjard et al., 1999, FEMS Microbiology Ecology, 0168-6496, Ranjard et al., 2000, Applied and Environmental Microbiology, vol. 66, pp 5334-5339 ; Ranjard et al., 2000, Microbial Ecology, vol. 39 (4), pp 263-272, Selenska-Pobell et al., 2001, Antonie van Leeuwenhoek, vol. 79 (2) pp 149-161).

Ces techniques fondées sur l'analyse de séquences génomiques utilisent en général, l'amplification PCR des acides nucléiques, afin d'obtenir une quantité suffisante de matériel génétique analysable.

Or, la technique d'amplification elle-même doit, pour être suffisamment sensible et quantitative, être mise en oeuvre à partir d'une quantité de matériel génétique extrait de l'échantillon suffisamment importante.

Cette quantité de matériel génétique extraite de l'échantillon dépend évidemment d'une part de la quantité de micro-organismes prélevés, et d'autre part du rendement de la méthode d'extraction d'acides nucléiques utilisée et du niveau de purification obtenu. Ainsi, il apparaît que la méthode d'extraction d'acides nucléiques est déterminante pour l'obtention de résultats fiables à partir d'un procédé d'analyse d'une population microbienne fondé sur l'analyse de séquences d'acides nucléiques extraits des micro-organismes d'un échantillon.

De nombreuses méthodes d'extraction d'acides nucléiques de micro-organismes sont connues de l'état de la technique. Ces méthodes comprennent généralement une étape de lyse, consistant à rompre la paroi et la membrane bactérienne ou fongique, une étape de précipitation des débris membranaires et protéiques, l'acide nucléique restant en solution, puis une étape de précipitation des acides nucléiques dans l'alcool, le cas échéant précédée d'une étape de purification des acides nucléiques en présence de phénol et de chloroforme. Ces techniques d'extraction sont bien connues de l'homme du métier et sont décrites en particulier dans Sambrook et al, 2001 (Molecular Cloning : A Laboratory Manual, 3rd Ed., Cold Spring Harbor, Laboratory Press, Cold Spring Harbor, New York). On citera en particulier les protocoles optimisés pour l'extraction d'ADN de micro-organismes prélevés dans l'environnement, tels que ceux décrits par Yeates et al., 1998 (Biological procedures, 1998, Vol. 1, No. 1, pages 40-47).

Cependant, ces techniques d'extraction s'avèrent encore insuffisantes en terme de rendement ou de qualité pour leurs utilisations dans certains procédés d'analyse microbienne.

En effet, si certains milieux environnementaux tels que l'eau ou le sol sont riches en micro-organismes, il n'en va pas de même pour l'analyse d'autres environnements tels que l'air, qui requiert un échantillonnage à partir de volumes très importants en particulier en raison de rendements d'extraction insuffisants. Des rendements d'extraction élevés sont en outre requis lorsque les micro-organismes prélevés sont sous la forme de spores, naturellement plus résistante aux étapes de lyse.

Il existe par conséquent un réel besoin de développer de nouvelles méthodes d'extraction d'acides nucléiques à rendement élevé, qui puissent être utilisables en routine dans des procédés d'analyse de population microbienne de l'environnement.

La première étape des méthodes d'extractions d'acides nucléiques consiste à lyser les membranes des micro-organismes. Les protocoles d'extractions connus dans l'état de la technique utilisent en alternative, trois modes de lyse : une lyse chimique à l'aide d'un détergent, une lyse mécanique par agitation en présence de billes ou une lyse par choc thermique par répétition de congélation et d'incubation à très haute température.

De telles méthodes ont été décrites notamment par Kuske et al., Applied And Environmental Microbiology, vol. 64, no. 7, juillet 1998, pages 2463-2472, par Giffin et al. Letters In Applied Microbiology, vol. 34, no. 3, 2002, pages 210-214, par Miller et al., Applied And Environmental Microbiology, vol. 65, no. 11, 1999, pages 4715-4724, par More et al., Applied And Environmental Microbiology, vol. 60, no. 5, 1er mai 1994, pages 1572-1580, par Odumeru et al., Canadian Journal of Veterinary Research, vol. 65, no. 4, octobre 2001, pages 201-205 et dans la demande de brevet EP-A-0 795 602 (BECTON DICKINSON CO), publiée le 17 septembre 1997.

Alors que les étapes de lyse par choc thermique ou de lyse mécanique sont généralement considérées comme des alternatives à la lyse chimique, il a maintenant été constaté, de manière surprenante, que la mise en oeuvre successive des trois étapes de lyse, chimique, mécanique et par choc thermique augmentait considérablement le rendement d'extraction, notamment en comparaison avec une combinaison de seulement deux des trois étapes de lyse.

Un premier objet de l'invention porte ainsi sur une méthode d'extraction d'acides nucléiques de micro-organismes, comprenant :
a. la mise en oeuvre successive des trois étapes de lyse suivantes dans un ordre quelconque :
   i. une lyse chimique des micro-organismes comprenant la mise au contact des micro-organismes avec une solution de lyse contenant un détergent ;
   ii. une lyse par choc thermique comprenant l'incubation de l'extrait à une température inférieure à 0°C, suivie immédiatement de l'incubation de l'extrait à une température d'au moins 95°C, de préférence environ 100°C ; et,
   iii. une lyse mécanique comprenant l'agitation de l'extrait en présence de billes.
b. la précipitation des débris membranaires et protéiques,
c. la récupération du surnageant contenant les acides nucléiques,
d. la précipitation des acides nucléiques et l'élimination du surnageant,
e. le cas échéant, la remise en solution des acides nucléiques dans un tampon approprié.

Selon la méthode de l'invention, les micro-organismes sont mis en suspension dans une solution contenant le tampon de lyse approprié pour la mise en oeuvre de l'une des trois étapes de lyse, chimique, mécanique ou thermique.

Dans le texte qui suit, on appellera « extrait », la solution contenant les acides nucléiques à extraire obtenue après l'une quelconque des étapes de la méthode d'extraction, de la mise en suspension des micro-organismes avant leur lyse jusqu'à l'extrait final obtenu.

On appellera « acides nucléiques », indifféremment, sauf précision spécifique, les acides désoxyribonucléiques (ADN), simple brin ou double brin, ou les acides ribonucléiques (ARN), incluant en particulier, les ARN messagers, les ARN ribosomaux et les ARN de transferts.

Au sens de l'invention, l'étape de lyse chimique comprend la mise au contact des micro-organismes en présence d'une solution contenant une quantité suffisante de détergent déterminée par l'homme du métier pour obtenir un rendement optimal. Des exemples de détergents utilisables pour la lyse chimique sont le CTAB ou le sodium dodecyl sulfate (SDS). Dans un mode de mise en oeuvre spécifique, on utilisera comme détergent, le SDS, par exemple à une concentration comprise entre 1 et 4%, classiquement environ 2%.

L'étape de lyse par choc thermique comprend l'incubation de l'extrait à une température inférieure à 0°C, suivi immédiatement de l'incubation de l'extrait à une température d'au moins 95°C, de préférence environ 100°C.

Les températures d'incubation seront choisies de sorte que la température de l'extrait atteigne une valeur inférieure à 0°C puis une valeur supérieure à 95°C. Elles sont par exemple comprises entre - 90°C et 0°C pour le point bas, de préférence compris entre - 80°C et - 60°C, et compris entre 95°C et 110°C pour le point haut, de préférence entre 95°C et 100°C. Le choc thermique signifie que la température de l'extrait passe d'une température inférieure à 0°C à une température supérieure à 95°C dans un temps inférieur à 1 minute, de préférence inférieur à 30 secondes, et mieux dans un temps inférieur à 1 seconde.

Classiquement, pour un volume d'extrait inférieur à 1 millilitre, l'extrait est incubé à une température inférieure à 0°C jusqu'à congélation puis incubé à une température supérieure à 95°C, de préférence environ 100°C pendant au moins 2 minutes, de préférence 3 minutes.

Pour congeler rapidement l'extrait, on pourra utiliser l'azote liquide ou tout autre gaz inerte liquide à une température très inférieure à 0°C.

Aussi, dans un mode de mise en oeuvre particulier, la lyse par choc thermique comprend l'incubation de l'extrait à une température d'environ - 70°C, par exemple dans de l'azote liquide, suivie immédiatement de l'incubation de l'extrait à une température d'au moins 95°C, de préférence environ 100°C, par exemple dans de l'eau bouillante.

Le choc thermique décrit ci-dessus peut être répété autant de fois que nécessaire, de préférence au moins trois fois, afin d'obtenir un rendement optimal.

L'étape de lyse pourra être précédée le cas échéant d'une lyse enzymatique afin de dégrader les protéines des parois microbiennes, par exemple en incubant les micro-organismes en suspension dans une solution contenant des protéases à une température appropriée. A titre d'exemples de protéase, on citera les protéinase K, classiquement utilisées dans les protocoles d'extraction d'acides nucléiques.

Selon l'invention, la lyse mécanique comprend l'agitation de l'extrait en présence de billes. La vitesse d'agitation, la taille des billes et la quantité de billes utilisées sont déterminées par l'homme du métier de façon à obtenir un fragilisation des membranes optimales. Les billes sont notamment des billes de verres de faible diamètre classiquement utilisées dans les protocoles d'extractions d'acides nucléiques par lyse mécanique, et notamment les protocoles d'extractions d'acides nucléiques de micro-organismes comportant une paroi rigide.

On peut en particulier procéder à l'agitation de l'extrait au moyen de broyeur, en présence de billes d'un diamètre compris entre 50 µm et 200 µm, de préférence compris entre 90 et 150 µm, par exemple environ 100 µm. Des billes de diamètres différents peuvent être combinées. En particulier, on utilisera des billes d'un diamètre compris entre 50 µm et 200 µm, à hauteur de 500 mg, par exemple entre 400 mg et 600 mg, pour un volume d'extrait compris entre 500 et 1500 µl, en combinaison avec quelques billes de plus gros diamètre, par exemple de 1 à 10 billes de diamètre compris entre 1 et 3 mm, par exemple 4 billes de 2 mm de diamètre.

De préférence, les trois étapes de lyse définies en (a) sont réalisées dans l'ordre suivant : (i) la lyse chimique, (ii) la lyse par choc thermique et (iii) la lyse mécanique.

Une fois le lysat obtenu, il est possible de procéder à nouveau à une dégradation enzymatique des protéines contenues dans le lysat, par exemple au moyen de protéases telles que les protéinase K.

Le surnageant contenant les acides nucléiques en solution est ensuite récupéré et les acides nucléiques sont précipités selon les méthodes classiquement utilisées, par exemple en présence d'un sel tel que l'acétate de sodium et d'alcool, notamment l'éthanol ou l'isopropanol. Le cas échéant, l'étape de précipitation pourra être suivie d'une étape de purification, par exemple en utilisant un mélange de phénol et de chloroforme.

Après précipitation, les acides nucléiques sont remis en solution dans un tampon approprié.

La méthode d'extraction des acides nucléiques est appropriée pour l'extraction d'acides nucléiques de tout type de population microbienne procaryote ou eucaryote inférieur, incluant, les bactéries, les protozoaires, les algues unicellulaires, les archaebactéries ou les champignons.

Plus particulièrement, la méthode de l'invention permet l'extraction d'acides nucléiques d'organismes microbiens qui sont principalement des bactéries et/ou des champignons.

Ces organismes microbiens peuvent comprendre notamment des spores de bactéries et/ou de champignons.

Dans un mode de mise en oeuvre particulier, les étapes de lyse sont précédées d'une étape de remise en culture des spores dans des conditions appropriées pour permettre d'initier la germination, par exemple en incubant les spores dans un milieu de culture riche à une température appropriée, classiquement entre 15 minutes et 3 heures à la température optimale de culture des formes végétatives des micro-organismes, par exemple entre 15 et 45 minutes à 37°C.

Les micro-organismes peuvent être prélevés de tout type d'environnement naturel, incluant le sol, l'eau ou l'air. En particulier, la méthode est appropriée pour des prélèvements de micro-organismes contenus dans l'air.

La méthode est également appropriée pour extraire des acides nucléiques à partir d'un nombre limité de micro-organismes, par exemple compris entre 10³ et 10⁸ CFU/ml, de préférence compris entre 10⁶ et 10⁷ CFU/ml.

Compte tenu des rendements importants obtenus par la méthode d'extraction d'acides nucléiques selon l'invention, ladite méthode est particulièrement appropriée pour son utilisation dans un procédé d'analyse de la population microbienne.

Un deuxième objet de l'invention porte donc sur un procédé d'analyse de la population microbienne d'un environnement, comprenant :
a. le prélèvement dans l'environnement d'un échantillon de la population microbienne, par exemple sous forme d'aérosol, et la remise en suspension du prélèvement dans une solution,
b. l'extraction des acides nucléiques de la population microbienne remise en suspension selon la méthode d'extraction de l'invention définie précédemment.
c. l'analyse des acides nucléiques extraits.

Dans un mode de mise en oeuvre spécifique du procédé, la population microbienne à analyser est prélevée dans l'air. En particulier, la population microbienne prélevée est une population bactérienne et/ou fongique.

Toute méthode d'analyse des acides nucléiques extraits peut être par la suite utilisée. De préférence, la méthode choisie doit permettre de caractériser la structure et la complexité de la communauté bactérienne ou fongique contenue dans le prélèvement.

Dans un exemple de mise en oeuvre du procédé, l'analyse des acides nucléiques extraits consiste à rechercher des marqueurs génétiques spécifiques permettant de caractériser la population microbienne, l'ensemble des marqueurs constituant l'empreinte génétique.

Une empreinte génétique est par exemple obtenue par l'amplification de fragments d'acides nucléiques spécifiques du génome d'espèces microbiologiques, notamment par l'amplification de fragments d'ARN ribosomiques, de préférence de l'espace intergénique 16S-23S des micro-organismes analysés, selon la technique RISA décrite par exemple dans Ranjard et al., 2001, Applied and Environmental Microbiology, oct 2001, pp 4479-4487.

Les exemples qui suivent permettent d'illustrer certains modes de mise en oeuvre spécifiques des méthodes de l'invention, sans toutefois en limiter la portée.

### LEGENDE DES FIGURES

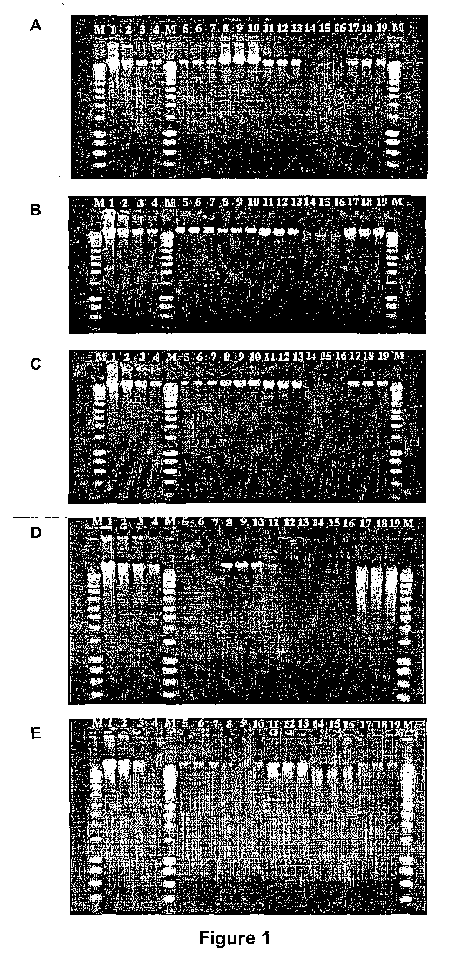

**Figure 1** **:** La figure 1 comprend des photos de gel d'agarose dans lesquels ont été migrés des extraits d'acides nucléiques.
A : Gel d'agarose de l'ADN extrait de la souche *Ralstonia metallidurans* CH34. Pistes 1-4 gamme étalon (500 ng, 250 ng, 124 ng, 62.5 ng respectivement); pistes 5-7 : lyse chimique ; pistes 8-10: lyse thermique ; pistes 11-13 : lyse mécanique 1600 rpm, pistes 14-16: lyse mécanique 3000 rpm, pistes 17-19 : kit BIO 101.
B : Gel d'agarose de l'ADN extrait de la souche *Bacillus polymixa* CFBP1954. Pistes 1-4 gamme étalon ; pistes 5-7 : lyse chimique ; pistes 8-10 : lyse thermique ; pistes 11-13 : lyse mécanique 1600 rpm, pistes 14-16 : lyse mécanique 3000 rpm, Pistes 17-19, kit BIO 101.
C : Gel d'agarose de l'ADN extrait de la souche *Pseudomonas fluorescens* C7R12. Pistes 1-4 : gamme étalon ; pistes 5-7 : lyse chimique ; piste 8-10 : lyse thermique ; pistes 11-13 : lyse mécanique 1600 rpm, pistes 14-16 : lyse mécanique 3000 rpm, pistes 17-19 : kit BIO 101.
D : Gel d'agarose de l'ADN extrait de la souche *Agrobacterium tumefaciens* C58. Pistes 1-4 : gamme étalon ; pistes 5-7 : kit BIO 101; pistes 8-10 : lyse chimique ; pistes 11-13: lyse thermique, pistes 14-16 : lyse mécanique 1600 rpm, pistes 17-19 : lyse mécanique 3000 rpm.
E : Gel d'agarose de l'ADN extrait de la souche *Rhodococcus.* Dépôt 20 µl. Pistes 1-4 : gamme étalon ; pistes 5-7 : lyse chimique ; pistes 8-10 : lyse thermique ; pistes 11-13 : lyse mécanique 1600 rpm, pistes 14-16 : lyse mécanique 3000 rpm, Pistes 17-19 : kit BIO 101.

**Figure 2** **:** La figure 2A est un histogramme illustrant les quantités d'ADN obtenues pour chaque souche avec les différents protocoles : chimique, thermique, mécanique 1600 rpm, mécanique 3000 rpm et Kit BIO101.

La figure 2B est un histogramme illustrant les quantités d'ADN obtenues pour chaque protocole avec les différentes souches. Les barres représentent les écarts-types.

### Agrobacterium, Ralstonia, Bacillus, Pseudomonas et Rhodococcus

**Figure 3** **:** La figure 3A est une photo d'un gel d'agarose sur lequel a été migré l'ADN extrait par une méthode combinant deux modes de lyse différents :
Pistes M : Marqueur de taille
Pistes 1-4 : Gamme étalon
Pistes 5-7 : *Agrobacterium* lyses mécanique+chimique
Pistes 8-10 : *Agrobacterium* lyses chimique+thermique
Pistes 11-13 : *Ralstonia* lyses mécanique+chimique
Pistes 14-16 : *Ralstonia* lyses chimique+thermique
Pistes 17-19 : *Bacillus* lyses mécanique+chimique
Pistes 20-22 : *Bacillus* lyses chimique+thermique
Pistes 23-25 : *Pseudomonas* lyses mécanique+chimique
Pistes 26-28 : *Pseudomonas* lyses chimique+thermique
Pistes 29-31 : *Rhodococcus* lyses mécanique+chimique
Pistes 32-34 : *Rhodococcus* chimique+thermique
Pistes 35-37 : spores lyses mécanique+chimique
Pistes 38-40 : spores lyses thermique+mécanique

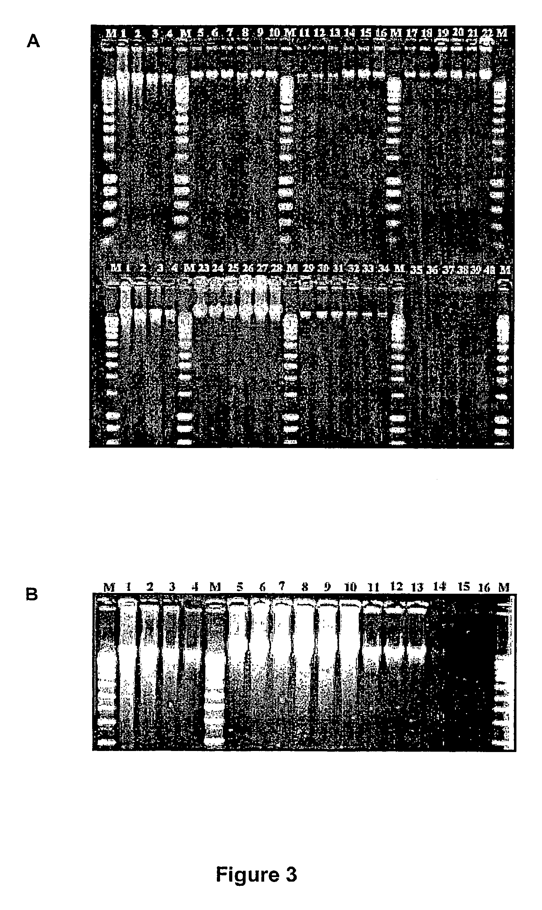

La figure 3B est une photo d'un gel d'agarose sur lequel a été migré l'ADN extrait par une méthode combinant les trois modes de lyse différents :
Pistes 1-4 : gamme étalon
Pistes 5-7 : *Agrobacterium*
Pistes 8-10 :*Pseudomonas*
Pistes 11-13 : *Rhodococcus*
Pistes 14-16 : Spores

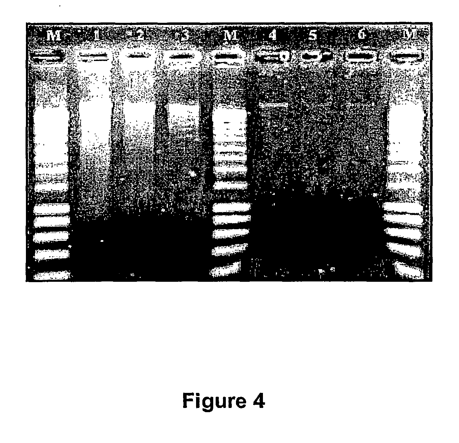

**Figure 4** **:** La figure 4 est un gel d'agarose sur lequel a été migré l'ADN extrait des spores de *Bacillus globigiil.*
Pistes M : Marqueur de tailles,
Pistes 1-3 : gamme étalon
Pistes 4-6 trois répétitions de dépôts de 30 µl

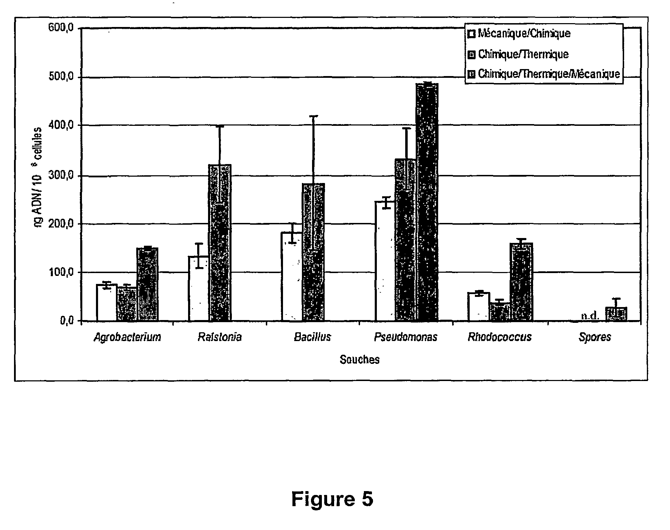

**Figure 5** **:** La figure 5 est un histogramme illustrant les quantités d'ADN obtenues pour chaque souche avec les différents protocoles combinant deux ou trois modes de lyses.
Mécanique+chimique, chimique+thermique,
chimique+thermique+mécanique.

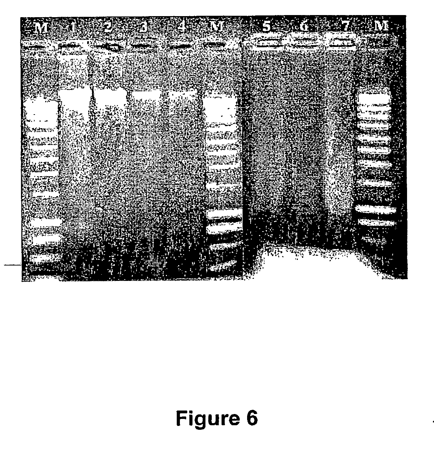

**Figure 6** **:** La figure 6 est un gel d'agarose sur lequel a été migré un ADN extrait de spores de *Bacillus globigii à* la concentration de 10⁹ spores/ml, dépôts de 30 µl.

Piste M : marqueur de taille ; pistes 1-4 : gamme étalon ; pistes 5-7 : 3 dépôts de 30 µl.

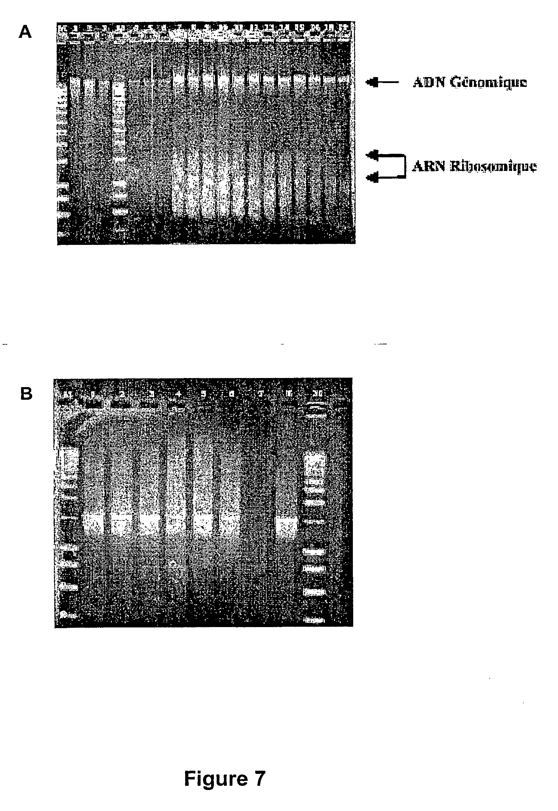

**Figure 7** **:** La figure 7A est un gel d'agarose sur lequel a été migré un ADN extrait de spores de *Bacillus globigii* souche speywood. Dépôts: 10 µl. Pistes M : Marqueur de taille ; Pistes 1-3, gamme étalon, pistes 4-6, sans remise en culture, pistes 7-9, +0,5h dans du TS à 37°C, pistes 10-12, +1 h dans du TS à 37°C ; pistes 13-15, +1,5h dans du TS à 37°C, pistes 16-19, +2h dans du TS à 37°C.

La figure 7B est un gel d'agarose sur lequel a été migré un ADN 16S de spores de *Bacillus globigii* souche speywood amplifié avec la *Taq* polymérase Qbiogene. Dépôts : 10 µl. Pistes M : Marqueur de taille ; Pistes 1-3, +0,5h dans du TS à 37°C, pistes 4-6, sans remise en culture ; piste 7, témoin négatif ; piste 8 témoin positif.

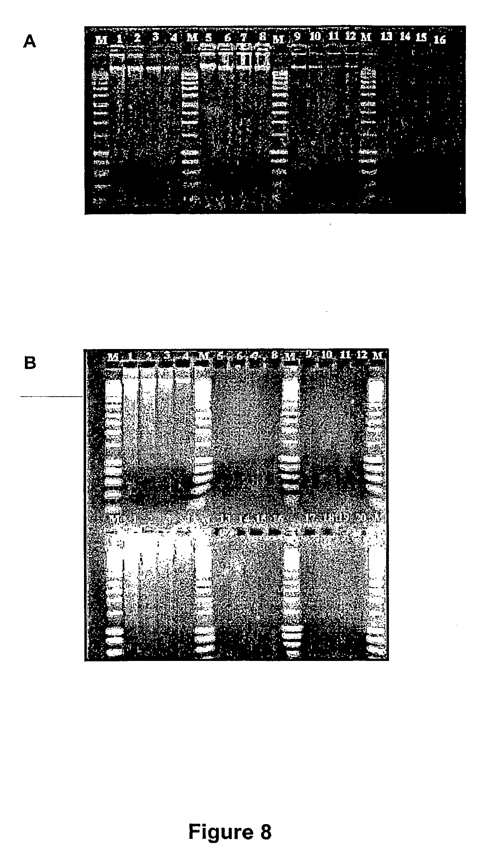

**Figure 8** **:** La figure 8A est un gel d'agarose sur lequel a été migré un ADN extrait de communautés reconstituées à différentes concentrations, avec la méthode- d'extraction de l'invention combinant les trois modes de lyse, dépôts de 10 µl.

Piste M : marqueur de taille ; pistes 1-4 : gamme étalon ; pistes 5-8 : 6.10⁸ CFU/mL ; pistes 9-12 : 6.10⁷ CFU/mL, pistes 13-16 : 6.10⁶CFU/mL.

La figure 8B est un gel d'agarose sur lequel a été migré un ADN extrait de communautés reconstituées à différentes concentrations, avec la méthode d'extraction de l'invention combinant les trois modes de lyse, dépôts de 20 µl.

Piste M : marqueur de taille ; pistes 1-4 : gamme étalon ; pistes 5-8 : 6.10⁵ CFU/mL ; pistes 9-12 : 6.10⁴ CFU/mL, pistes 13-16 : 6.10³CFU/mL, pistes 17-20 : 6.10² CFU/mL.

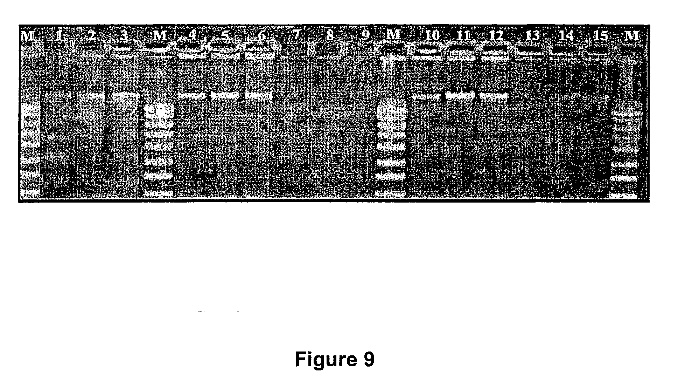

**Figure 9** **:** La figure 9 est un gel d'agarose sur lequel a été migré un ADN extrait de spores et des formes végétatives de *Bacillus globigii* (souche CIP 7718 et souche speywood) avec la méthode d'extraction de l'invention combinant les trois modes de lyse, dépôts de 20 µl.

Piste M : marqueur de taille ; pistes 1-3 : gamme étalon ; pistes 4-6 : formes végétatives de la souche ClP7718 ; pistes 7-9 : formes sporulées de la souche CIP7718, pistes 10-12: formes végétatives de la souche speywood ; pistes 13-15 : formes sporulées de la souche speywood.

### EXEMPLES

Cinq souches représentatives des grands groupes bactériens ont été utilisées ainsi que des formes sporulées afin de comparer les rendements d'extrait obtenus au moyen d'un kit commercial d'extraction d'acides nucléiques et de différents protocoles basés sur les principes d'une lyse chimique, d'une lyse thermique et d'une lyse mécanique. Après ce premier criblage, un éventuel effet synergique de l'association de plusieurs protocoles a été testé, ainsi qu'une optimisation de l'étape de germination des formes sporulées. La méthode d'extraction de l'invention a ensuite été mise en oeuvre à partir de communautés bactériennes dites "reconstituées" à différentes concentrations.

### A/ Solutions, Souches, Conditions de cultures et titration

**Milieu LB :**
   Bacto-tryptone 10 g
   NaCl 5 g
   Extrait de levure 10 g
   H₂O qsp 1 L
   *+ Agar 15 g*/*L*
**Milieu B de King :**
   Biogélitone 20 g
   Glycérol 10 ml
   KH2PO4 1.5 g
   MgSO4, 7H₂O 1.5 g
   H₂O: qsp 1 litre
   *+ Agar 15 g*/*L*

### Préparation du Tampon d'extraction de l'ADN TES+SDS à 2%

### Préparation avec + tween 20 à 0,01% : TWEEN 20 1 mL + 999 mL

### Tris-HCl 1M - pH 8

Pour 500 mL :
60,57 g Tris
+ 25 mL HCl pour ajuster à pH 8
+ H₂O qsp 500 mL
*Stérilisation* : Autoclavage
*Stockage* : Température ambiante

### SDS 20 %

Pour 500 mL :
100 g dans environ 400 mL d'H₂O puis chauffer pour dissoudre. + H₂O qsp 500 mL
*Stérilisation* : filtration mais pas nécessaire
*Stockage* : Température ambiante

### NaCl 1M

Pour 500 mL :
29,22 g de NaCl
+ H₂O qsp 500 mL
*Stérilisation* : Autoclavage
*Stockage* : Température ambiante

### EDTA 0,5M - pH8

Pour 500 mL : 12
93,085 g EDTA
+ 400 mL H₂O
+ Ajuster à pH 8 avec NaOH concentré (EDTA soluble à pH 8)
+ H₂O qsp 500 mL
*Stérilisation* : Autoclavage
*Stockage* : Température ambiante

### Tampon d'extraction (Concentrations finales):

TRIS HCl 100 mM pH8, EDTA 100mM pH 8, NaCl 100mM, 2% SDS.

Donc pour 100 mL de Tampon : 10 mL de TRIS 1M, 20 mL EDTA 0,5M, 10mL NaCl 1M, 10 mL SDS 20 %, + 50 mL

### Acétate de Potassium 3M - pH 5,5

Pour 100mL :
60 mL Acétate de potassium 5M
+ 11,5 mL Acide Acétique 100 %
+ H₂O qsp 100 mL
*Stérilisation* : filtration
*Stockage* : Température ambiante

**Les souches bactériennes utilisées** sont représentatives des grands types bactériens :
- *Alpha-protéobactérie* : *Agrobacterium tumefaciens* C58
- Beta-protéobactérie : *Ralstonia metallidurans* CH34
- Gama-protéobactérie : *Pseudomonas fluorescens* C7R12
- Gram + à Bas GC : *Bacillus polymyxa* CFBP1954 (Collection Française des Bactéries Phytopathogènes)
- Gram + à Haut GC : *Rhodococcus rhodochrous* ATCC 13898
- Spores de *Bacillus globigii* souche CIP 7718 : 5 mL de suspension dans de l'eau + TWEEN 20 à 0,01%, à une concentration de 10⁹ spores.ml⁻¹ et utilisées lors de l'extraction à une concentration de 10⁸ spores.ml⁻¹.

Les bactéries *Ralstonia, Pseudomonas, Bacillus, Rhodococcus* sont cultivées en milieux Luria-Bertoni (LB) et *Agrobacterium* en milieu King B. A la densité optique de 1 (600 nm), des suspensions, dilutions et étalements de chaque souche sont réalisés sur les milieux appropriés (LB agar et King B agar). La titration des souches est effectuée par dénombrement des colonies après incubation des boites à 28°C pendant 4 jours. A cette même densité optique, des aliquotes de 1 mL de cultures sont prélevés, centrifugés (8000g, 15 minutes) et congelés (-20°C) pour les étapes futures d'extraction d'ADN. Cette étape permet d'éviter la possible lyse des cellules bactériennes dans le milieu de culture lors de la congélation.

Les dénombrements obten us sont présentés dans le tableau 1 suivant. A la densité optique de longueur d'ondes à 600 nm égale à 1, toutes les souches sont comprises entre 3,7.10⁸ et 1,6.10⁹ CFU.ml⁻¹, densité suffisante pour l'extraction d'ADN.

**Tableau 1 : Titration des souches**

| **Souches** | **CFU/mL** | **+/-** | **écart-type** |
|---|---|---|---|
| Agrobacterium tumefaciens C58 | 1,60.10⁹ | +/- | 0,49.10⁸ |
| Bacillus polymyxa CFBP 1954 | 4,62.10⁸ | +/- | 7,07.10⁷ |
| Pseudomonas fluorescens C7R12 | 6,85.10⁸ | +/- | 1,20.10⁸ |
| Ralstonia metallidurans CH 34 | 3,76.10⁸ | +/- | 0,94.10⁷ |
| Rhodococcus Rhodochrous ATCC13898. | 8,30.10⁸ | +/- | 0,52.10⁸ |

### B/ Extraction d'ADN sur souches

### 1/ Protocoles séparés

Les ADN de 5 souches bactériennes ainsi que de spores sont extraits au moyen des protocoles d'extraction décrits dans l'exemple B. Pour chaque échantillon, 3 répétitions ont été effectuées. Les protocoles utilisés sont basés sur les grands principes d'extraction d'ADN couramment utilisés en écologie moléculaire. Il s'agit (i) d'une lyse mécanique fondée sur l'action de billes de verres fragilisant les membranes bactériennes, (ii) une lyse chimique basée sur l'action d'un détergent, le sodium dodecyl-sulfate (SDS) pour fragiliser les membranes et enfin (iii) une lyse thermique reposant sur l'alternance de chocs chauds et froids pour faire éclater les cellules.

Le protocole de lyse mécanique comprend les premières étapes suivantes:
- A partir du culot Bacterien.
- Reprendre avec 1 ml de tampon d'extraction TES (fait avec H₂O + tween 20 à 0.01%) (Tris-HCl 100 mM pH 8, EDTA 100 mM pH 8, NaCl 100 mM).
- Ajouter 500 mg de billes de 106 µm lavées et 4 billes de 2 mm de diamètre (pratiquement peser les billes dans les tubes adaptés puis ajouter le tampon+culot).
- Agiter les tubes dans le broyeur à 1600 rpm pendant 30 secondes ou 3000 rpm 1 minute (support préalablement placé à -20°C).
- Centrifuger 14 000g, 2 minutes (culoter les billes) puis récupérer le surnageant.

Le protocole de lyse thermique comprend les premières étapes suivantes :
- A patir du culot bactérien.
- Reprendre avec 1 ml de tampon d'extraction TES (fait avec H₂O + tween20 à 0.01%) (Tris-HCl 100 mM pH 8, EDTA 100 mM pH 8, NaCl 100mM).
- 3 cycles de « Heat shock » :
   Azote liquide (jusqu'à congélation) → Eau bouillante (3 minutes à 100 °C).

Le protocole de lyse chimique comprend les premières étapes suivantes :
- A partir du culot bactérien.
- Reprendre avec 1 ml de tampon d'extraction TES (fait avec H₂O + tween 20 à 0.01%) + SDS 2 % (Tris-HCl 100 mM pH 8, EDTA 100 mM pH 8, NaCl 100 mM, 2% SDS).
- Incubation à 70°C pendant 30 minutes en « vortexant » 10 secondes après 15 et 30 minutes.

Les dernières étapes sont communes à tous les protocoles. Elles consistent tout d'abord à l'ajout de protéinase K pour dénaturer les protéines, puis à l'incubation avec de l'acétate de potassium sur la glace afin de précipiter et d'éliminer les protéines après centrifugation (14000g, 5 min). L'ADN est enfin précipité par de l'isopropanol froid (v/v).

Le protocole des étapes communes est détaillé ci-après :
- Ajouter Proteinase K : 20µL d'une Solution Mère à 10mg/mL.
- Incuber 30 minutes entre 37 et 50 °C.
- Ajouter 1/10 du volume (environ 100 µL) d'acétate de Potassium 3M pH 5,5.
- Incuber 10 minutes sur la glace.
- Centrifuger à 14 000 g pendant 5 minutes et récupérer le surnageant dans un tube de 2 mL.
- Ajouter 1 volume d'isopropanol à -20°C.
- Placer 30 minutes à -20°C.
- Centrifuger à 13 000 rpm pendant 30 minutes.
- Eliminer le surnageant avec précaution avec une pipette.
- Laver le culot à l'éthanol 70° (200 µl) à -20°C (sans remettre l'ADN en suspension). Centrifuger 5 minutes à 13 000 rpm.
- Eliminer l'alcool et laisser sécher le culot 30 minutes à température ambiante (ou à l'étuve) jusqu'à ce qu'il n'y ait plus de traces d'alcool sur les parois du tube
- Reprendre le culot dans 100 µl de H₂O.

Les protocoles de préparation des différents tampons ont été décrits précédemment.

Le kit commercial d'extraction d'ADN FASTDNA^{®} KIT (référencé dans le texte par KIT BIO101) est quant à lui une combinaison de la lyse mécanique et de la lyse chimique. Le protocole élaboré par le fournisseur Qbiogene est décrit dans la note technique fournie avec le KIT.

L'ADN extrait de chaque souche bactérienne est quantifié sur gel par comparaison avec une gamme étalon d'ADN de thymus de veau. 10µL d'ADN extrait sont déposés sur un gel d'agarose à 1%. Une gamme étalon d'ADN de thymus de veau correspondant à 500 ng ADN/10µL, 250 ng ADN/10µL, 125 ng ADN/10µL, 62,5 ng ADN/10µL est également déposée. Après migration et coloration au bromure d'éthidium, le gel est traité par un analyseur d'image permettant de calculer une droite étalon et *in fine* de déterminer les quantités d'ADN extraites pour chaque souche.

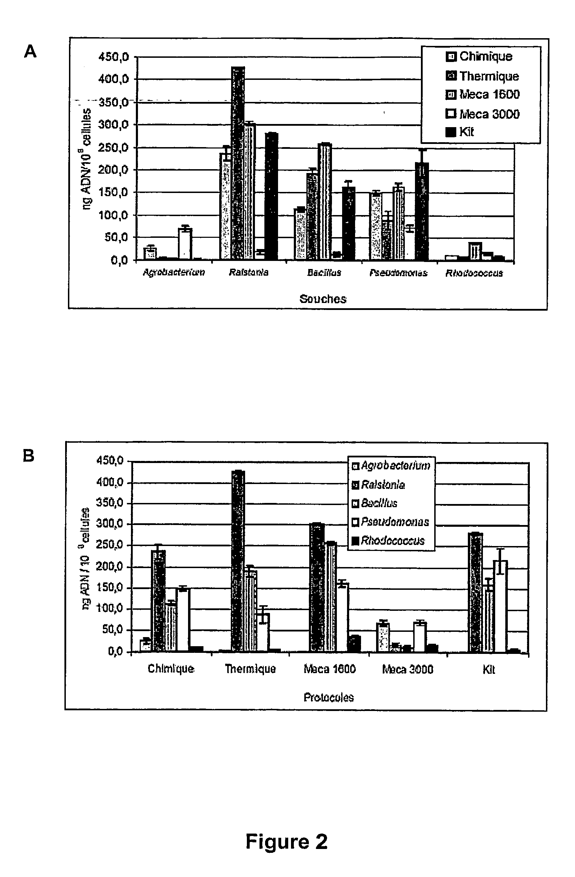

Les figures 1A à 1E représentent les dépôts de 10 µL d'ADN pour *Agrobacterium, Pseudomonas, Bacillus, Ralstonia* et 20 µL pour *Rhodococcus* respectivement. Le gel correspondant aux spores n'a pas été présenté car aucun ADN n'était détectable visuellement. On remarque dès à présent sur ces photos, (i) l'effet négatif de la lyse mécanique à 3000 rpm pendant 1 minute sur l'intégrité de l'ADN extrait et (ii) l'extrême hétérogénéité des rendements d'ADN entre les protocoles. Cette observation est confirmée par le traitement informatique de l'image. Les quantités d'ADN, ramenées à 10⁸ CFU.ml⁻¹, sont rapportées dans le tableau 2 suivant et présentées en fonction des souches sur la figure 2A et en fonction des protocoles sur la figure 2B. Les quantités d'ADN sont comprises entre 0,9 et 425 ng d'ADN /10⁸ CFU.

**Tableau 2 : Quantité d'ADN pour 10⁸ CFU extrait de chaque souche avec les différents protocole :**

| Ng ADN /10⁸ CFU | | Agrobacterium | Agrobacterium | Ralstonia | Bacillus | Pseudomonas |
|---|---|---|---|---|---|---|
| Chimique | moy. | 26.0 | 236,1 | 113,3 | 149,4 | 10,8 |
| | ecart-type | 5,4 | 16,4 | 5,7 | 5,8 | 0,4 |
| Thermique | moy. | 3,5 | 425,2 | 190,7 | 89,0 | 6,6 |
| | ecart-type | 1,8 | 3,2 | 12,9 | 20,4 | 0,3 |
| Meca 1600 | moy. | 1,6 | 302,4 | 257,5 | 162,7 | 38,0 |
| | ecart-type | 1,3 | 4,1 | 3,9 | 9,0 | 0,9 |
| Meca 3000 | moy. | 69,4 | 17,8 | 12,0 | 71,6 | 16,1 |
| | ecart-type | 7,0 | 4,0 | 4,5 | 7,1 | 2,8 |
| kit | moy. | 0,9 | 280,5 | 160,6 | 216,2 | 7,7 |
| | ecart-type | 0,3 | 2,8 | 14,8 | 29,0 | 2,0 |

Les principaux enseignements de ces résultats sont que (i) aucun des protocoles ne semble adapté à une extraction équivalente, en terme de rendement, pour toutes les souches testées et (ii) outre le kit, la lyse mécanique à 1600 rpm pendant 30 secondes, la lyse chimique et la lyse thermique semblent donner les meilleurs résultats.

### 2/ Synergie des protocoles de lyse sur les rendements d'extraction

### a/ Synergie de deux protocoles

Afin d'augmenter les quantités d'ADN extrait mais également de niveler les différences entre les souches, un possible effet synergique des protocoles d'extraction a été évalué. Pour cela, les protocoles qui donnaient les résultats les plus significatifs ont été combinés de manière indépendante.

Les associations lyse chimique / lyse mécanique à 1600 rpm pendant 30 secondes, et lyse chimique / lyse thermique ont été testées.

La figure 3A correspond aux dépôts de 10 µL d'ADN pour *Agrobacterium, Pseudomonas, Bacillus, Ralstonia* et 20 µL pour *Rhodococcus* et les spores. Cependant, aucun ADN n'est observé pour les spores, comme indiqué sur les pistes 35 à 40. Les quantités données en figure 5 sont comprises entre 37,4 et 332,4 ng d'ADN/10⁸ CFU. On observe un effet synergique sur les souches d'*Agrobacterium* et de *Rhodococcus,* sans pour autant montrer de différences significatives entre les 2 combinaisons différentes.

Pour les souches de *Bacillus, Ralstonia* et de *Pseudomonas,* on note une uniformisation de la quantité d'ADN pour les deux combinaisons. Cette quantité est plus élevée pour la combinaison lyse chimique + thermique avec néanmoins une variabilité importante entre les répétitions d'une même souche (cf. écart-types, figure 5).

### b/ Synergie de la combinaison des trois modes de lyse

Une combinaison des trois protocoles de lyse a également été testée pour évaluer un éventuel effet synergique, c'est-à-dire la combinaison d'une lyse chimique puis thermique et enfin mécanique, avec une étape préalable à la protéinase K (fragilisation des membranes cellulaires) et une vitesse de 1600 rpm pendant 1 minute. Le protocole détaillé de cette synergie est présenté ci-après :
- A partir du culot Batérien
- Reprendre avec 1ml de tampon d'extraction TES (fait avec H₂O + tween 20 à 0,01%) + SDS 2% (Tris-HCl 100 mM pH 8, EDTA 100 mM pH 8,NaCl 100 mM, 2% SDS).
- Peser 500 mg de billes de 106 µm lavées et 4 billes de 2 mm de diamètre (pratiquement peser les billes dans les tubes adaptés puis ajouter le tampon + culot).
- Ajouter Proteinase K : 20 µL d'une Solution Mère à 10mg/mL.
- Incuber 30 minutes à 37°C.
- Incubation à 65°C pendant 30 minutes en (vortexant) 10 secondes après 15 et 30 minutes. (Chimique)
- 3 cycles de « Heat shock » :
   Azote liquide (jusqu'à congélation) → Eau bouillante (3 minutes à 100°C). (thermique)
- Agiter les tubes dans le broyeur à 1600 rpm pendant 1 minutes (support préalablement placé à -20 °C). (Mécanique)
- Centrifuger à 14 000 g pendant 1 minute et récupérer le surnageant dans un tube de 2 ml
- Ajouter 1/10 du volume (environ 100 µL) d'acétate de Potassium 3M pH 5,5.
- Incuber 10 minutes sur la glace.
- Centrifuger à 14 000g pendant 5 minutes et récupérer le surnageant dans un tube de 2 mL.
- Ajouter 1 volume d'isopropanol préalablement placé à -20°C.
- Incuber 30 minutes à -20 °C.
- Centrifuger à 13 000 rpm pendant 30 minutes.
- Eliminer le surnageant avec précaution à l'aide d'une pipette.
- Laver le culot à l'éthanol 70° (200µl) à -20 °C (sans remettre l'ADN en suspension). Centrifuger 5 minutes à 13 000 rpm.
- Eliminer l'alcool et, laisser sécher le culot 30 minutes à température ambiante (ou à l'étuve) jusqu'à ce qu'il n'y ait plus de traces d'alcool sur les parois du tube.
- Reprendre le culot dans 100 µl de H₂O.

Cette combinaison de trois protocoles de lyse a été utilisée pour lyser les spores de *Bacillus globigii* et deux souches (*Agrobacterium* et *Rhodococcus*) dont les rendements d'extraction sont les plus faibles ainsi que la souche de *Pseudomonas,* utilisée comme témoin d'extraction.

La figure 3B représente les dépôts de 10 µL d'ADN pour *Pseudomonas, Rhodococcus Agrobacterium* et les spores. Les résultats des quantités d'ADN sont présentés sur la figure 5. On remarque d'emblée la faiblesse des écart-types mais également le fort effet synergique de cette technique. Les quantités d'ADN sont respectivement doublées et triplées, par rapport à la synergie de deux modes de lyse, pour *Agrobacterium* et *Rhodococcus.* De plus, cette synergie permet d'extraire de l'ADN à partir de spores (*B. globigii* souche CIP 7718) à hauteur de 30 ng ADN/10⁸ spores. En effet, le dépôt de 30 µL (figure 4) de l'ADN extrait des spores permet de visualiser un ADN de haut poids moléculaire.

Dans le but de confirmer ces résultats sur les spores, le protocole d'extraction combinant les trois modes de lyse a été mis en oeuvre sur 10⁹ spores. La figure 6 montre une quantité non négligeable d'ADN extrait des spores. En effet, après quantification l'ADN extrait a été estimé à 1080 ng pour les 10⁹ spores, soit un rendement de 24% d'efficacité pour l'extraction.

La combinaison de la lyse chimique, de la lyse thermique et de la lyse mécanique, est donc adaptée à l'extraction d'acides nucléiques de micro-organismes représentatifs des grands phylums et produit un effet synergique sur les rendements d'extraction obtenus. Les principaux avantages de cette synergie sont donc :
(i) une quantité d'ADN extrait sur toutes les souches suffisantes pour l'amplification par PCR
(ii) une bonne reproductibilité et enfin
(iii) la possibilité d'extraire de l'ADN sur les spores, éléments hautement résistants et potentiellement présents à forte densité dans l'air.

### C/ Rendement d'extraction.

Les rendements d'extraction ont été calculés avec la taille du génome des souches utilisées, obtenue à partir de bases de données disponibles (http://www.ncbi.nlm.nih.gov/PMGifs/Genomes/eub_g.html), et en sachant que 1 Mb correspond à 1.10⁻¹⁵ g d'ADN. Connaissant les quantités d'ADN extrait en ng /10⁸ CFU, il est ainsi possible de déterminer des rendements qui sont compris entre 10 % pour les spores et 75 % pour *Pseudomonas* (avec la combinaison de trois protocoles de lyse). Cependant, la moyenne de ces rendements, si l'on excepte celui obtenu pour les spores, se situe aux alentours de 50 %.
Les rendements d'extraction d'ADN obtenues par combinaison des 2 ou 3 modes de lyses et estimées pour chaque souche par rapport à la taille du génome sont indiqués dans le tableau 3 suivant :

**Tableau 3 : Rendement d'extraction d'ADN estimée pour chaque souche par rapport à la taille du génome.**

| Souches | Tailles génome (Mb) | ngADN/10⁸ CFU | Rendement (%) |
|---|---|---|---|
| Agrobacterium tymefaciens C58* | 5,66 | 149,7 | 26,5 |
| Bacilus polymyxa CFBP 1954 ** | 4,5 | 282,5 | 62,8 |
| Pseudomanas fluorescens C7R12 * | 6,47 | 484,4 | 74,8 |
| Ralstonia metallidurans CH 34 ** | 6,76 | 321,5 | 47,6 |
| Rhodococcus sp. * | 6,5 | 157,8 | 24,6 |
| Spores 10⁸ cellules * | 4,5 | 47,7 | 10,4 |
| Spores 10⁸ cellules * | 4,5 | 1080/10⁹ cellules | 24 |

| | | | |
|---|---|---|---|
| ** Quantité obtenue avec la synergie de 2 protocoles * Quantité obtenue avec la synergie de 3 protocoles | | | |

### D/ Extraction sur "communautés reconstituées"

Afin de déterminer la limite de sensibilité de la méthode d'extraction selon l'invention, l'ADN de communautés "reconstituées" a été extrait à différentes concentrations selon la méthode d'extraction de l'invention combinant les trois modes de lyse.

Pour cela, 10⁸ CFU des 5 souches bactériennes ainsi que des formes sporulées (*B. globigii* souche CIP 7718) ont été remis en suspension dans 1 millilitre de solution. Cette suspension à la concentration de 6.10⁸ CFU.ml⁻¹ a servi de solution mère à des dilutions au dixième jusqu'à 6.10² CFU.ml⁻¹. Quatre répétitions par dilution ont été utilisées pour l'extraction d'ADN. Les résultats sont présentés sur les figures 8A et 8B avec des dépôts de 10 µL pour les concentrations de 6.10⁸ à 6.10⁶ CFU.ml⁻¹ et des dépôts de 20µL de 6.10⁵ à 6.10². La quantité d'ADN obtenue pour 6.10⁸ est d'environ 3000 ng avec un rendement de 82 % pour une taille de génome moyen de 5,5 Mb. A la densité de 6.10⁷ CFU.ml⁻¹ la quantité d'ADN diminue environ d'un facteur 10 pour atteindre 400 ng. Pour les fortes dilutions (de 6.10⁶ à 6.10² CFU.ml⁻¹), l'ADN extrait n'est pas visible. Un calcul théorique aboutit à une concentration de 4 ng d'ADN pour 10µL déposé à une densité cellulaire de 6.10⁶ CFU.ml⁻¹. Une telle quantité n'est pas visible sur gel d'agarose et coloration au bromure d'éthidium, ce qui explique ces résultats. La méthode d'extraction combinant les trois modes de lyse permet ainsi d'extraire de façon très satisfaisante les ADN de communautés reconstituées avec des rendements relativement élevés.

### E/ Test d'extraction d'ADN sur des spores d'une autre souche de Bacillus globigii : la souche speywood.

Les résultats obtenus montrent que les spores sont récalcitrantes à la lyse. En effet même en utilisant la méthode d'extraction de l'invention, les rendements d'extraction d'ADN restent faibles. Pour améliorer ces rendements, un protocole incluant une étape préalable de remise en culture des formes sporulées en milieu liquide TS (trypticase soy) pendant 2 heures à 37°C a été testé. Cette incubation permet le passage des formes sporulées aux formes végétatives. Les ADN des échantillons ont ensuite été extraits à l'aide de la méthode d'extraction de l'invention. Ce protocole a été testé sur deux souches de *Bacillus globigii* : les souches speywood (aérosolisable) et CIP 7718 (non aérosolisable).

L'ADN obtenu à partir des formes végétatives et sporulées des deux souches de *Bacillus globigii* a été déposé sur gel et les résultats sont présentés en figure 9. Les quantifications correspondant à ces dépôts sont données dans le tableau 4 suivant.

**Tableau 4**

| | Souches | Qté ADN (ng / 10⁸ spores) | Moyenne (ng / 10⁸ spores) | Rendement Théorique (%) | Effet sporulation (%) |
|---|---|---|---|---|---|
| Formes Végétatives | CIP 1 | 135,6 | 193,3 | 43,0 | 3,3 |
| (non aérosolisables) | CIP 2 | 210,9 | | | |
| | CIP 3 | 233,5 | | | |
| Formes sporulées | CIP 4 | 12,0 | 6,3 | 1,4 | |
| (non aérosolisables) | CIP 5 | 7,4 | | | |
| | CIP 6 | -0,6 | | | |
| Formes Végétatives | Spey 1 | 106,6 | 172,9 | 38,4 | 16,1 |
| (aérosolisables) | Spey 2 | 224,1 | | | |
| | Spey 3 | 188,0 | | | |
| Formes sporulées | Spey 4 | 21,4 | 27,8 | 6,2 | |
| (aérosolisables) | Spey 5 | 31,1 | | | |
| | Spey 6 | 30,9 | | | |

Les résultats obtenus montrent que le passage de la forme sporulée à la forme végétative permet une augmentation de la quantité d'ADN extrait quelle que soit la souche considérée (de 6 à 193 ng d'ADN/10⁸ spores de la souche CIP et de 28 à 173 ng d'ADN/10⁸ spores pour la souche speywood).

Considérant que le temps de génération de la majorité des bactéries de l'environnement est supérieur à 1 heure, l'étape d'incubation de 2 heures à 37°C ne devrait pas modifier la composition et la densité de la communauté bactérienne des échantillons. Cette étape pourrait donc s'avérer intéressante dans l'optique d'une extraction efficace de l'ADN des spores contenues dans les échantillons d'air.

### F/Effet de la remise en culture sur le rendement d'extraction et d'amplification de l'ADN des formes sporulées

Les formes sporulées pouvant potentiellement représenter une partie importante du bruit de fond biologique de l'air, il était apparu important de rechercher un moyen d'améliorer ce rendement. Au cours de la première étape l'effet de la germination sur le rendement d'extraction de l'ADN à partir d'échantillons de formes sporulées avait été testé en incubant les spores de deux souches CIP7718 et speywood de *Bacillus globigii* à 37°C dans du milieu de culture (TS) pendant 2h. Cette étape d'incubation était destinée à induire le passage des formes sporulées à des formes végétatives plus facilement lysable. Les cellules avaient été ensuite lysées et l'ADN extrait déposé sur gel. Les résultats obtenus montraient que pour les deux souches testées, l'étape d'incubation améliorait de façon significative le rendement d'extraction de l'ADN des formes sporulées.

Dans l'hypothèse où les formes sporulées pourraient représenter une partie importante du bruit de fond biologique de l'air, cette étape d'incubation semble donc recommandée. Cependant, elle doit être d'une durée suffisamment courte pour éviter la croissance des populations bactériennes sous formes végétatives également présentes dans les échantillons. C'est pourquoi il a été effectué une cinétique de germination des spores afin de déterminer le temps d'incubation minimum suffisant pour améliorer le rendement d'extraction de l'ADN. Pour cela, les formes sporulées de la souche speywood de *B. glogigii* ont été incubées à 37°C dans du milieu TS pendant 0,5 ; 1 ; 1,5 et 2h.

L'ADN a ensuite été extrait en utilisant le protocole de synergie par 3 et déposé sur gel (Figure 7A). Les résultats obtenus montrent que 30 minutes d'incubation suffisent pour améliorer significativement le rendement d'extraction de l'ADN à partir des formes sporulées. Cette incubation est suffisamment courte pour qu'aucune modification de la densité cellulaire des échantillons n'intervienne et que l'équilibre relatif des différentes populations bactériennes ne soit biaisé.

Par la suite, nous avons effectué l'amplification par PCR du gène 16S de l'ADN extrait des formes sporulées après 0 et 0,5 heure d'incubation. Les résultats obtenus sont présentés dans la Figure 7B. Les profils montrent que l'amplification est efficace sur les formes sporulées après 0 et 0,5 heures de remise en culture dans du milieu TS à 37°C.

Cependant, il est apparu que cette étape d'incubation améliore de façon significative le rendement et la reproductibilité de l'extraction et de l'amplification de l'ADN des spores.

### G/ Analyse des extraits d'ADN de populations microbiennes prélevées dans l'air

Afin d'étudier des communautés microbiennes prélevés dans l'air par des outils de biologie moléculaire, les extraits d'acides nucléique obtenus selon la méthode d'extraction de l'invention sont analysés par réalisation d'une empreinte génétique de la population par la technique RISA (Ribosomal Intergenic Spacer Analysis) telle que décrite dans Ranjard et al., 2001, Applied and Environmental Microbiology, pp 4479-4487, ou la T-RFLP (Terminal-Restriction Fragment Length Polymorphism) (Moeseneder et al., 2001, Journal of microbiological methods, Vol 44 (2) pp 159-172 ; Saka et al., Soil Science and Plant Nutrition, Vol 47 (4) 773-778 ; Urakawa et al., 2000, MEPS, 220 : 47-57 ; Marsh, 1999, Current opinion in Microbiology, Vol 2 (3) 323-327.

## Revendications

1. Méthode d'extraction d'acides nucléiques de micro-organismes, comprenant
a. la mise en oeuvre successive des trois étapes de lyse suivantes dans un ordre quelconque :
i. une lyse chimique des micro-organismes comprenant la mise au contact des micro-organismes avec une solution de lyse contenant un détergent ;
ii. une lyse par choc thermique comprenant l'incubation de l'extrait à une température inférieure à 0°C, suivie immédiatement de l'incubation de l'extrait à une température d'au moins 95°C, de préférence environ 100°C ; et,
iii. une lyse mécanique comprenant l'agitation de l'extrait en présence de billes.
b. la précipitation des débris membranaires et protéiques,
c. la récupération du surnageant contenant les acides nucléiques,
d. la précipitation des acides nucléiques et l'élimination du surnageant,
e. le cas-échéant la remise en solution des acides nucléiques dans un tampon approprié.

2. Méthode selon la revendication 1, **caractérisée en ce que** les trois étapes de lyse définies en (a) sont réalisées dans l'ordre suivant : (i) la lyse chimique, (ii) la lyse par choc thermique et (iii) la lyse mécanique.

3. Méthode selon la revendication 1 ou 2, **caractérisée en ce que** la lyse chimique comprend l'utilisation d'une solution de lyse comprenant un détergent, de préférence du SDS (sodium dodecyl sulfate), de manière encore plus préférée, du SDS à une concentration comprise entre 1 et 4%.

4. Méthode selon l'une des revendications 1 à 3, **caractérisée en ce que** la lyse comprend l'incubation de l'extrait à une température d'environ - 70°C, par exemple dans de l'azote liquide, suivie immédiatement de l'incubation de l'extrait à une température d'au moins 95°C, de préférence environ 100°C, par exemple dans de l'eau bouillante.

5. Méthode selon l'une quelconque des revendications 1 à 4, **caractérisée en ce que** la lyse mécanique comprend une agitation dans un broyeur en présence de billes d'un diamètre compris en 50 µm et 200 µm, de préférence d'environ 100 µm.

6. Méthode selon l'une quelconque des revendications 1 à 5, **caractérisée en ce que** les microorganismes sont essentiellement des bactéries et/ou des champignons.

7. Méthode selon l'une quelconque des revendications 1 à 6, **caractérisée en ce que** les microorganismes peuvent être sous forme de spores de bactéries et/ou de champignons.

8. Méthode selon la revendication 7, **caractérisée en ce que** les étapes de lyse sont précédées d'une étape de remise en culture des spores, comprenant la culture des spores dans un milieu riche approprié pour initier la germination.

9. Méthode selon l'une quelconque des revendications précédentes, **caractérisée en ce que** les organismes microbiologiques sont prélevés dans l'air.

10. Procédé d'analyse de la population microbienne d'un environnement, comprenant
a. le prélèvement dans l'environnement d'un échantillon de la population microbienne et la remise en suspension du prélèvement dans une solution,
b. l'extraction des acides nucléiques de la population microbienne remise en suspension selon la méthode définie à l'une quelconque des revendications 1 à 9,
c. l'analyse des acides nucléiques extraits.

11. Procédé selon la revendication 10, **caractérisé en ce que** la population microbienne à analyser est prélevée dans l'air.

12. Procédé selon la revendication 10 ou 11, **caractérisé en ce que** la population microbienne prélevée est une population bactérienne et/ou fongique.

13. Procédé selon l'une quelconque des revendications 10 à 12, **caractérisé en ce que** l'analyse des acides nucléiques extraits consiste à rechercher des marqueurs génétiques permettant de caractériser la population microbienne, l'ensemble des marqueurs constituant l'empreinte génétique.

14. Procédé selon la revendication 13, **caractérisé en ce qu'**une empreinte génétique est obtenue par l'amplification de fragments d'acides nucléiques spécifiques du génome d'espèces microbiennes, notamment par l'amplification de fragments d'ARN ribosomiques, de préférence de l'espace intergénique 16S-23S des micro-organismes analysés.

## Claims

1. Method for extracting nucleic acids from microorganisms, comprising:
a. successively implementing the following three lysis steps in any order:
i. chemical lysis of the microorganisms, comprising bringing the microorganisms into contact with a lysis solution containing a detergent;
ii. lysis by thermal shock, comprising incubating the extract at a temperature below 0°C, and then immediately incubating the extract at a temperature of at least 95°C, preferably approximately 100°C; and
iii. mechanical lysis, comprising agitating the extract in the presence of beads,
b. precipitating the membrane and protein debris,
c. recovering the supernatant containing the nucleic acids,
d. precipitating the nucleic acids and removing the supernatant,
e. where appropriate, resolubilizing the nucleic acids in a suitable buffer.

2. Method according to Claim 1, **characterized in that** the three lysis steps defined in (a) are carried out in the following order: (i) chemical lysis, (ii) lysis by thermal shock and (iii) mechanical lysis.

3. Method according to Claim 1 or 2, **characterized in that** the chemical lysis comprises the use of a lysis solution comprising a detergent, preferably SDS (sodium dodecyl sulphate), even more preferably SDS at a concentration of between 1% and 4%.

4. Method according to one of Claims 1 to 3, **characterized in that** the lysis comprises incubating the extract at a temperature of approximately -70°C, for example in liquid nitrogen, and then immediately incubating the extract at a temperature of at least 95°C, preferably approximately 100°C, for example in boiling water.

5. Method according to any one of Claims 1 to 4, **characterized in that** the mechanical lysis comprises agitation in a mill in the presence of beads having a diameter of between 50 µm and 200 µm, preferably of approximately 100 µm.

6. Method according to any one of Claims 1 to 5, **characterized in that** the microorganisms are essentially bacteria and/or fungi.

7. Method according to any one of Claims 1 to 6, **characterized in that** the microorganisms may be in the form of bacterial and/or fungal spores.

8. Method according to Claim 7, **characterized in that** the lysis steps are preceded by a step of placing the spores back in culture, comprising culturing the spores in a suitable rich medium in order to initiate germination.

9. Method according to any one of the preceding claims, **characterized in that** the microbiological organisms are taken from the air.

10. Method for analysising the microbial population of an environment, comprising:
a. taking a sample of the microbial population from the environment and resuspending the sample in a solution,
b. extracting the nucleic acids from the resuspended microbial population according to the method defined in any one of Claims 1 to 9,
c. analysing the extracted nucleic acids.

11. Method according to Claim 10, **characterized in that** the microbial population to be analysed is taken from the air.

12. Method according to Claim 10 or 11, **characterized in that** the microbial population taken is a bacterial and/or fungal population.

13. Method according to any one of Claims 10 to 12, **characterized in that** the analysis of the extracted nucleic acids consists in searching for genetic markers for characterizing the microbial population, the collection of markers constituting the genetic fingerprint.

14. Method according to Claim 13, **characterized in that** a genetic fingerprint is obtained by amplifying fragments of nucleic acids specific for the genome of microbial species, in particular by amplifying ribosomal RNA fragments, preferably the 16S-23S intergenic space of the microorganisms analysed.

## Patentansprüche

1. Verfahren zur Extraktion von Nukleinsäuren aus Mikroorganismen, umfassend:
a. Ausführen der folgenden drei Lyseschritte in einer beliebigen Reihenfolge nacheinander:
i. eine chemische Lyse von Mikroorganismen umfassend das in Kontakt bringen der Mikroorganismen mit einer Lyselösung, die einen oberflächenaktiven Stoff enthält,
ii. eine Lyse durch Thermoschock umfassend eine Inkubation des Extrakts bei einer Temperatur unter 0 °C, unmittelbar gefolgt von einer Inkubation des Extrakts bei einer Temperatur von mindestens 95 °C, bevorzugt ungefähr 100 °C, und
iii. eine mechanische Lyse umfassend Durchmischen des Extrakts in Gegenwart von Kugeln,
b. Ausfällen von Membran- und Proteinbruchstücken,
c. Aufnahme des Überstands, der die Nukleinsäuren enthält,
d. Ausfällen der Nukleinsäuren und Entfernen des Überstands,
e. gegebenenfalls in Lösung bringen der Nukleinsäuren in einem geeigneten Puffer.

2. Verfahren nach Anspruch 1, **dadurch gekennzeichnet, dass** die in (a) definierten drei Lyseschritte in der folgenden Reihenfolge ausgeführt werden: (i) chemische Lyse, (ii) Lyse durch Thermoschock und (iii) mechanische Lyse.

3. Verfahren nach Anspruch 1 oder 2, **dadurch gekennzeichnet, dass** die chemische Lyse Verwendung einer Lyselösung umfasst, die einen oberflächenaktiven Stoff enthält, bevorzugt SDS (Natriumdodecylsulfat), besonders bevorzugt SDS in einer Konzentration zwischen 1 und 4 %.

4. Verfahren nach einem der Ansprüche 1 bis 3, **dadurch gekennzeichnet, dass** die Lyse die Inkubation des Extrakts bei einer Temperatur von ungefähr -70 °C, zum Beispiel in flüssigem Stickstoff, unmittelbar gefolgt von der Inkubation des Extrakts bei einer Temperatur von mindestens 95 °C, bevorzugt ungefähr 100 °C, zum Beispiel in siedendem Wasser umfasst.

5. Verfahren nach einem der Ansprüche 1 bis 4, **dadurch gekennzeichnet, dass** die mechanische Lyse eine Durchmischung in einer Mühle in Gegenwart von Kugeln mit einem Durchmesser zwischen 50 µm und 200 µm, bevorzugt ungefähr 100 µm umfasst.

6. Verfahren nach einem der Ansprüche 1 bis 5, **dadurch gekennzeichnet, dass** die Mikroorganismen im Wesentlichen Bakterien und/oder Pilze sind.

7. Verfahren nach einem der Ansprüche 1 bis 6, **dadurch gekennzeichnet, dass** die Mikroorganismen in Form von Sporen von Bakterien und/oder Pilzen vorliegen können.

8. Verfahren nach Anspruch 7, **dadurch gekennzeichnet, dass** den Lyseschritten ein Schritt zur Kultivierung von Sporen vorausgeht, der die Kultur von Sporen in einem geeigneten angereicherten Milieu umfasst, um die Keimung auszulösen.

9. Verfahren nach einem der vorhergehenden Ansprüche, **dadurch gekennzeichnet, dass** die mikrobiologischen Organismen aus der Luft entnommen sind.

10. Analyseverfahren für die Mikrobenpopulation einer Umgebung, umfassend:
a. Entnehmen einer Probe der Mikrobenpopulation aus der Umgebung und in Suspension bringen der Entnahme in einer Lösung,
b. Extraktion von Nukleinsäuren der Mikrobenpopulation in Suspension nach dem Verfahren, wie es in einem der Ansprüche 1 bis 9 definiert ist,
c. Analyse der extrahierten Nukleinsäuren.

11. Analyseverfahren nach Anspruch 10, **dadurch gekennzeichnet, dass** die zu analysierende Mikrobenpopulation aus der Luft entnommen ist.

12. Analyseverfahren nach Anspruch 10 oder 11, **dadurch gekennzeichnet, dass** die entnommene Mikrobenpopulation eine Population von Bakterien und/oder Pilzen ist.

13. Analyseverfahren nach einem der Ansprüche 10 bis 12, **dadurch gekennzeichnet, dass** die Analyse der extrahierten Nukleinsäuren Suchen nach genetischen Markern umfasst, die eine Charakterisierung der Mikrobenpopulation ermöglichen, wobei die Marker zusammen einen genetischen Fingerabdruck bilden.

14. Analyseverfahren nach Anspruch 13, **dadurch gekennzeichnet, dass** ein genetischer Fingerabdruck durch Amplifizierung von spezifischen Nukleinsäurefragmenten des Genoms von Mikrobenspezies erhalten wird, insbesondere durch Amplifizierung von Fragmenten von Ribosomen-RNA, bevorzugt des intergenischen Raums 16S-23S der analysierten Mikroorganismen.
